# EUROPÄISCHE PATENTANMELDUNG

(11) **EP 0 722 746 A1**
(43) Veröffentlichungstag der Anmeldung: **24.07.1996**
(21) Anmeldenummer: 95118325.0
(22) Anmeldetag: 22.11.1995
(51) Int. Cl.: A61M 15/00

(54) **Vorrichtung zum Erzeugen eines Aerosols aus einer pulverförmigen Substanz**

(30) Priorität: 21.01.1995 DE 19501831
(71) Anmelder: KÖHLER, Dieter, Prof. Dr. med., D-57392 Schmallenberg-Winkhausen (DE)
(72) Erfinder: KÖHLER, Dieter, Prof. Dr. med., D-57392 Schmallenberg-Winkhausen (DE)
(74) Vertreter: Lempert, Jost, Dipl.-Phys. Dr. rer.nat.

(57) **Zusammenfassung**

Die Erfindung betrifft eine Vorrichtung zur Erzeugung eines Aerosols aus einer pulverförmigen Substanz, insbesondere zur Inhalation, mit einem die zu verwirbelnde Substanz aufnehmenden Verwirbelungsraum, in dem ein Unterdruck erzeugbar ist, in den durch eine verschließbare Öffnung ein Gas zum Verwirbeln der Substanz und zur Erzeugung eines Aerosols einbringbar und aus dem durch eine verschließbare Öffnung eine vorbestimmte Menge des Aerosols ausbringbar ist. Zur Verbesserung der Ausbringungskonstanz ist vorgesehen, daß im Verwirbelungsraum (2) ein flexibles Element (5) aus elektrisch leitendem Material angeordnet ist.

## Beschreibung

Die Erfindung betrifft eine Vorrichtung zur Erzeugung eines Aerosols aus einer pulverförmigen Substanz, insbesondere zur Inhalation, mit einem die zu verwirbelnde Substanz aufnehmenden Verwirbelungsraum, in dem ein Unterdruck erzeugbar ist, in den durch eine verschließbare Öffnung ein Gas zum Verwirbeln der Substanz und zur Erzeugung eines Aerosols einbringbar und aus dem durch eine verschließbare Öffnung eine vorbestimmte Menge des Aerosols ausbringbar ist.

Zur Behandlung von Erkrankungen der Atemwege werden vorzugsweise Arzneistoffe mittels Inhalation appliziert, weil dadurch der Wirkstoff in relativ hoher Konzentration auf den Atemwegen deponiert wird, während Effekte infolge der Verteilung des Arzneistoffs im ganzen Körpers, sogenannte systemische Effekte, relativ gering bleiben. Dessen ungeachtet eignet sich die Inhalation eines Arzneistoffes auch zur systemischen Therapie.

Die Herstellung eines geeigneten Aerosols mit wirkungsvoll inhalierbaren, d.h. relativ kleinen Partikeln, bereitet zum Teil erhebliche Schwierigkeiten, insbesondere wenn von einer pulverförmigen Substanz ausgegangen wird. Aus diesem Grunde sind zahlreiche Vorrichtungen entwickelt worden, die auf unterschiedliche Art versuchen, ein solches gut inhalierbares Aerosol herzustellen.

Eine derartige gattungsgemäße Vorrichtung ist aus der PCT/EP93/03033 bekannt. Bei dieser Vorrichtung wird die pulverförmige Substanz in einem definierten, abgeschlossenen Verwirbelungsraum durch Einströmen eines Gases verwirbelt. Hierzu weist die Vorrichtung innerhalb eines Zylinders eine flexible Hülle auf, welche am Zylinder sowie an einem innerhalb des Zylinders zur Volumenänderung des in der Hülle ausgebildeten Verwirbelungsraums verschiebbaren Kolbens festgelegt ist. Auf der dem Kolben gegenüberliegenden Seite des Zylinders ist eine durch ein Verschlußorgan verschließbare Öffnung angeordnet. Durch diese Öffnung erfolgt das Einströmen des Gases sowie das Ausbringen des so erzeugten Aerosols. Das Verschlußteil für die Austrittsöffnung des Verwirbelungsraumes ist mit dem darin bewegbaren Kolben durch einen Faden verbunden. Um die im Verwirbelungsraum befindliche Substanz zu verwirbeln, wird zunächst ein Unterdruck erzeugt, indem der Kolben bei verschlossener Öffnung aus dem Zylinder zurückgezogen wird. Nach Durchlaufen einer vorgegebenen Strecke wird über den Faden die Öffnung durch weiteres Herunterziehen des Kolbens geöffnet und Luft kann in das Innere des Verwirbelungsraumes einströmen, wodurch die Substanz zur Erzeugung eines Aerosols verwirbelt wird. Durch Einschieben des Kolbens in den Zylinder wird dann das Aerosol aus dem Verwirbelungsraum heraus und in den Innenraum eines Mundstücks gedrückt. Aufgrund weiterer Einrichtungen kann der Patient oder Benutzer das Aerosol erst nach Schließen der Austrittsöffnung aus dem Mundstück einatmen. Durch diese Vorrichtung ist ein Aerosol mit einem hohen inhalierbaren Wirkstoffanteil gegeben. Um bei jeder Anwendung eine möglichst gleichgroße Deponierung der Wirkstoffe auf den Atemwegen zu erreichen, ist eine möglichst geringe Mengenvariation erforderlich.

Der Erfindung liegt daher die Aufgabe zugrunde, eine Vorrichtung der eingangs genannten Art mit einer verbesserten Variationsbreite zu schaffen.

Ausgehend von der Vorrichtung der eingangs genannten Art wird diese Aufgabe dadurch gelöst, daß im Verwirbelungsraum ein flexibles Element aus elektrisch leitendem Material angeordnet ist. Auf diese Weise können geladene Aerosolteilchen, welche ansonsten größere Agglomerate bilden, ihre Ladungsträger an das Material abgeben. Die Ladungsträger werden von diesem dann bei entsprechender Erdung abgeleitet, so daß durch die Reduktion der Ladungsträger im Verwirbelungsraum ein möglichst feldfreier Raum erzeugt wird. Ist ein solcher feldfreier Raum vorhanden, dann liegt das zerstäubte Pulver in einer außerordentlich hohen Konzentration mit einer stabilen Aerosoldichte vor. Aufgrund dieser hohen Aerosoldichte wird dann die Grenzkonzentration erreicht, die das zur Verwirbelung dienende Gas, insbesondere Luft, noch tragen kann. Lediglich noch vorhandene gröbere Partikel fallen rasch aus, und es ergibt sich eine stabile Aerosoldichte mit etwa gleichem Partikelspektrum. Die Mengenvariation bei den einzelnen Inhalationsvorgängen ist dabei bedeutend geringer als bei bekannten Vorrichtungen.

Durch die Verwendung eines flexiblen Elementes kann sich dieses gegebenenfalls bei entsprechender Anordnung der Vergrößerung bzw. Verringerung des Verwirbelungsraumvolumens anpassen, so daß jeweils im gesamten Verwirbelungsraum eine antistatische Fläche zur Abgabe der Ladungen vorhanden ist.

Hierzu trägt in Weiterbildung bei, daß sich das flexible Element aus elektrisch leitendem Material über den gesamten Querschnitt des Verwirbelungsraumes erstreckt.

Bei dem verwendeten elektrisch leitenden Material handelt es sich bevorzugt um ein Metall, wenn auch antistatisches Kunststoffmaterial einsetzbar ist. Eine bevorzugte Ausgestaltung sieht vor, daß das Element in Form eines Drahtknäuels ausgebildet ist. Es handelt sich um ein lockeres Drahtknäuel mit einer chaotischen Struktur der Drahtführung. Bevorzugt ist dabei vorgesehen, daß das Element in Form einer mehrfach gewundenen Schraubendrahtfeder oder aber einer räumlich auseinandergezogenen Drahtspirale mit konischer oder doppelkonischer Kontur ausgebildet ist. Aufgrund einer solchen Ausbildung steht im Verwirbelungsraum ein elektrisch leitendes Material großer Oberfläche zur Verfügung, an das die Aerosolpartikel ihre Ladungen abgeben können. Entsprechend ergibt sich, insbesondere in Verbindung mit Metallen hoher elektrischer Leitfähigkeit und einer Erstreckung des Elements über den gesamten Querschnitt des Verwirbelungsraumes eine optimale Abführung der Ladungen aus dem gesamten Verwirbelungsraum heraus.

Für den das flexible Element aus elektrisch leitendem Material enthaltenden Verwirbelungsraum ist bevorzugt vorgesehen, daß der Verwirbelungsraum zur Erzeugung des Unterdrucks in ihm vergrößert wird. Hierzu ist der Verwirbelungsraum in einer ersten Ausführungsform von einer Anordnung aus einem Kolben und einem Zylinder gebildet, bei der der Verwirbelungsraum durch Verschieben des Kolbens gegenüber dem Zylinder vergrößerbar ist. Damit nun das flexible Element aus elektrisch leitendem Material der Bewegung des Kolbens zur Vergrößerung des Verwirbelungsraumes bzw. zur Verkleinerung desselben zum Ausbringen des erzeugten Aerosols folgen kann, ist bevorzugt vorgesehen, daß das flexible Element aus elektrisch leitendem Material an der dem Verwirbelungsraum zugewandten Stirnseite des Kolbens an diesem sowie an dem der Stirnseite gegenüberliegenden Ende des Verwirbelungsraums an der Wandung des Zylinders festgelegt ist. Aufgrund dieser bevorzugten Ausgestaltung wird der Verwirbelungsraum jederzeit von dem elektrisch leitenden Material durchsetzt. Des weiteren befindet sich das Material aufgrund dieser Ausgestaltung bei Einströmen des zur Verwirbelung dienenden Gases direkt im Hauptstrom desselben, so daß jeweils eine direkte Kontaktmöglichkeit mit dem Ladungen aufweisenden Aerosolpartikel besteht. Des weiteren wird so aufgrund der hohen Strömungsgeschwindigkeiten im Verwirbelungsraum eine Adhäsion der Aerosolteilchen am elektrisch leitenden Material vermieden. Gleichzeitig wird durch dieses Material die Adhäsion der Teilchen an den Wänden des Verwirbelungsraumes verringert, indem während der Bewegung des Kolbens durch das den Querschnitt des Verwirbelungsraumes ausfüllende Element, insbesondere bei Verwendung einer mehrfach gewundenen Schraubendrahtfeder oder einer räumlich auseinandergezogenen Drahtspirale mit konischer oder doppelkonischer Kontur, ein Abkratzen von an der Wand haftenden Partikeln erfolgt. Diese Reinigungsfunktion des Elements trägt insbesondere dazu bei, daß nicht ein Teil des Pulvers durch Anhaften an den Wänden im Totwasser der Strömung der Verfügbarkeit zur Aerosolbildung entzogen wird. Das von den Wänden entfernte Material steht dann bei einem folgenden Inhalationsprozeß wieder zur Erzeugung von Aerosolen zur Verfügung.

In einer anderen Ausgestaltung ist vorgesehen, daß der Verwirbelungsraum durch einen an seinen Stirnseiten jeweils mit Deckplatten versehenen Faltenbalg gebildet und durch Ausdehnen des Faltenbalges vergrößerbar ist. Um auch hier die gleichen Effekte wie bei Verwendung eines Zylinders mit Kolben zu erzielen, ist das flexible Element aus elektrisch leitendem Material an den Deckplatten des Faltenbalges festgelegt. Entsprechend weist eine derartig ausgebildete Vorrichtung die gleichen Vorteile wie die vorher genannte auf.

Durch die Erfindung ist eine Vorrichtung gegeben, mittels welcher aufgrund der Ableitung der elektrischen Ladung der Aerosolteilchen eine deutliche Verbesserung der Variationsbreite und damit die Erzeugung eines hohen inhalierbaren Wirkstoffanteils bei einer guten Dosierkonstanz möglich ist.

Gemäß einer weiteren Ausbildung ist vorgesehen, daß Bereiche des Elements zumindest an der Umfangswandung des Verwirbelungsraums anliegen. Die an der Umfangswandung anliegenden Bereiche des Elements streifen bei Bewegung des Kolbens an dieser entlang und kratzen dabei dort haftendes Pulvermaterial ab.

Weitere Vorteile und Merkmale der Erfindung ergeben sich aus der nachfolgenden Beschreibung, in welcher Ausführungsbeispiele anhand der beiliegenden Zeichnungen näher erläutert sind. Dabei zeigt:
- Fig. 1: ein erstes vereinfachtes Ausführungsbeispiel der erfindungsgemäßen Vorrichtung;
- Fig. 2A: einen Längsschnitt durch eine zweite bevorzugte Ausführungsform der erfindungsgemäßen Vorrichtung mit verschlossener Öffnung; und
- Fig. 2B: den Längsschnitt der Fig. 2A bei offener Öffnung.

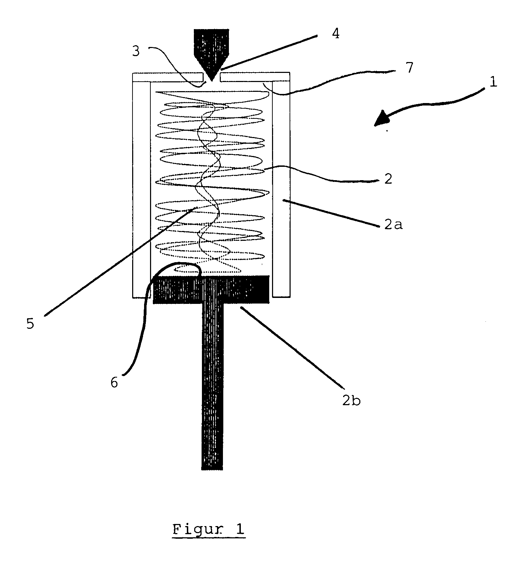

Das in Fig. 1 dargestellte erste vereinfachte Ausführungsbeispiel der Vorrichtung 1 weist eine Anordnung aus einem Zylinder 2a und einem Kolben 2b auf, welche gemeinsam einen Verwirbelungsraum 2 festlegen. Der Kolben 2b ist im Zylinder 2a beweglich angeordnet und zwischen einer vorgeschobenen und einer zurückgeschobenen Stellung hin- und herbewegbar. Die Größe des Verwirbelungsraumes ergibt sich somit aus der jeweiligen Position des Kolbens 2b. Des weiteren weist der Verwirbelungsraum 2 eine Öffnung 3 auf, durch die ein die im Verwirbelungsraum 2 vorhandene pulverförmige Substanz (in Fig. 1 nicht dargestellt) verwirbelndes Gas, vorzugsweise Umgebungsluft, einströmt und durch die dann das nach erfolgter Verwirbelung erzeugte Aerosol herausgedrückt wird. Damit der Verwirbelungsraum 2 bei der Herstellung des definierten Unterdrucks, z.B. des Vakuums, abgeschlossen ist, ist ein Verschlußorgan 4 für die Öffnung 3 vorgesehen.

Anstelle einer Öffnung 3 können aber auch für das Einströmen der Luft in den evakuierten Verwirbelungsraum 2 sowie für das Herausdrücken des Aerosols und gegebenenfalls auch zum Einbringen der pulverförmigen Substanz in den Verwirbelungsraum 2 jeweils getrennte Öffnungen vorgesehen sein.

Im Inneren des Verwirbelungsraums 2 ist eine Drahtschraubenfeder 5 aus Metall angeordnet. Die Schraubenfeder 5 ist mehrfach, hier zweifach, ineinander verwunden (Fig. 1). Die Außenwindungen haben eine den Innenabmessungen des Verwirbelungsraums 2 entsprechende Kontur. Die Schraubenfeder 5 ist dabei am Kolben 2b an dessen der Öffnung 3 zugewandten Stirnseite 6 sowie an dem mit der Öffnung 3 versehenen Boden 7 des Zylinders 2a festgelegt. Aufgrund dieser Festlegung folgt die Schraubenfeder 5 jeweils der Bewegung des Kolbens 2a zwischen der vorgeschobenen und zurückgeschobenen Stellung. Hierdurch steht jeweils bei jeder Position des Kolbens 2b über den gesamten Verwirbelungsraum 2 die Oberfläche der Schraubenfeder zur Reduktion der Ladungsträger sowie Erzeugung eines feldfreien Raums zur Verfügung.

Um eine Beeinträchtigung der Bewegbarkeit des Kolbens 2b im Zylinder 2a auszuschließen, die durch Pulver zwischen Zylinder und Kolben auftreten kann, kann im Inneren des Zylinders 2a ein zusätzlicher flexibler Behälter, etwa ein Faltenbalg oder ein dünnwandiger Kunststoffschlauch, zur Aufnahme der Substanz und entsprechend auch zur Aufnahme der Schraubenfeder 5 vorgesehen werden. Beim Erweitern des Verwirbelungsraumes, d.h. beim Verschieben des Kolbens 2b in die in Fig. 1 dargestellte zurückgezogene Stellung, erweitert sich dann ein solcher flexibler Behälter aufgrund des außen anliegenden Vakuums. Vorzugsweise kann der flexible Behälter auch am Kolben 2a befestigt sein, so daß er mit diesem jeweils mitbewegt wird.

Ein solcher Faltenbalg kann aber auch an seinen Stirnseiten mittels Deckplatten abgeschlossen sein, wobei dann die Schraubenfeder an diesen Deckplatten festgelegt ist.

Die Funktion dieses Ausführungsbeispiels der erfindungsgemäßen Vorrichtung ist folgendermaßen:
Entweder wird zu Beginn eine zu verwirbelnde pulverförmige Substanz in den Verwirbelungsraum 2 eingebracht oder aber sie ist in diesem bereits beinhaltet. Als nächstes wird dann im Verwirbelungsraum ein definiertes Vakuum erzeugt, in dem der Kolben 2b aus einer vorgeschobenen Stellung in der Nähe des Bodens 7 in die in Fig. 1 dargestellte zurückgeschobene Stellung gebracht wird. Dabei ist die Austrittsöffnung 3 des Zylinders 2a durch das Verschlußorgan 4 verschlossen. Bei der Erzeugung des Vakuums im Inneren des die Substanz enthaltenden Verwirbelungsraums 2 bleibt diese im wesentlichen unbeeinflußt. Die Schraubenfeder 5 folgt dabei der Bewegung des Kolbens 2b, wobei gegebenenfalls bei einem vorhergehenden Inhalationsvorgang an den Wänden des Zylinders 2a haftende Partikel durch die Schraubenfeder 5 von den Wänden entfernt werden.

Ist das definierte Vakuum erzeugt, dann wird einem Gas, vorzugsweise der Umgebungsluft, gestattet, rasch, aber kontrolliert in den die Substanz enthaltenden Verwirbelungsraum einzuströmen. Es wird also ein Luftstrom erzeugt, der in den Verwirbelungsraum rasch, aber kontrolliert einströmt. Dies erfolgt dabei durch Öffnen des Verschlußorgans 4 an der Austrittsöffnung 3. Durch den Luftstrom wird die pulverförmige Substanz verwirbelt, und es erfolgt eine gute Verteilung der Partikel in dem zur Verfügung stehenden Verwirbelungsraum 2, so daß das gewünschte Aerosol erzeugt wird und den Verwirbelungsraum nahezu vollständig ausfüllt. Die Verteilung der Partikel stellt sich unter sehr genau definierbaren Bedingungen ein, da durch die Größe und Gestalt des zur Verfügung stehenden Verwirbelungsraumes 2 und der Öffnung 3, durch die der Luftstrom in den Verwirbelungsraum gelangt, sowie durch deren Lage das Strömungsverhalten und damit die Verwirbelung der pulverförmigen Substanz in starkem Maße bestimmt werden kann. Vorteilhaft dabei ist, daß sich die Schraubenfeder 5 direkt im Hauptstrom der einströmenden Luft befindet. Hierdurch können während der Verwirbelung geladene Aerosolteilchen diese Ladung an die Schraubenfeder abgeben. Des weiteren wird aufgrund der starken Strömung verhindert, daß die Aerosolteilchen an der Schraubenfeder 5 haften bleiben.

Als nächstes wird dann das erzeugte Aerosol aus dem Verwirbelungsraum 2 ausgebracht und dadurch dem Patienten zur Inhalation bereitgestellt. Dies kann beispielsweise durch einfaches Herausdrücken des Aerosols erfolgen, indem der Kolben 2b wieder in seine vorgeschobene Stellung gebracht wird. Während dieses Vorganges wird dann auch die Schraubenfeder 5 wiederum zusammengedrückt, wobei auch hier gleichzeitig eine Reinigung der Zylinderwände einschließlich Deck- und Bodenfläche erfolgt.

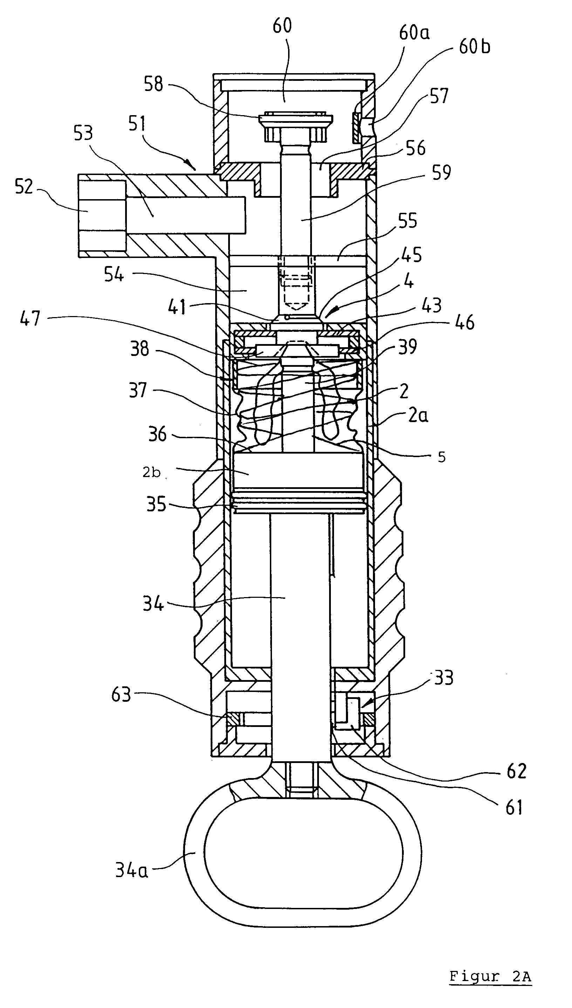

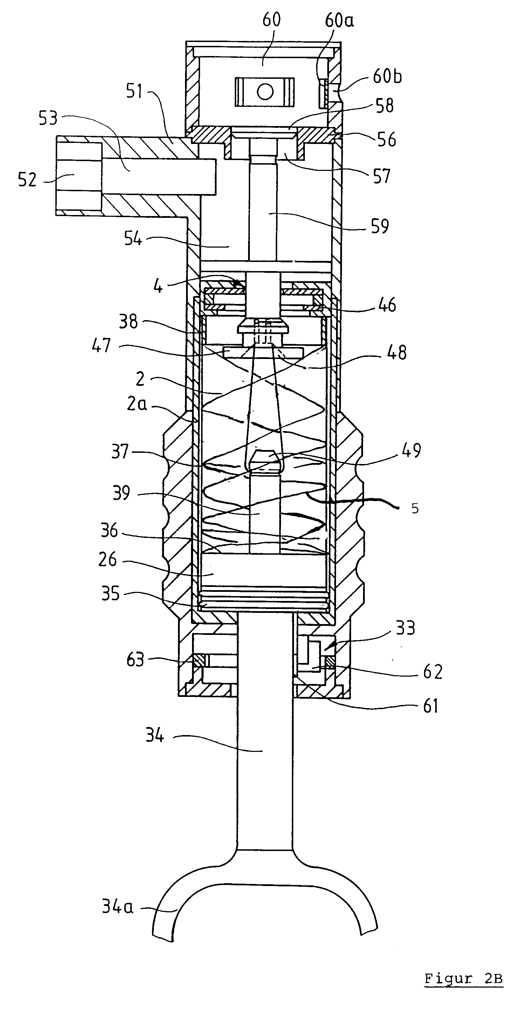

In den Fig. 2A und 2B ist eine weitere bevorzugte Ausführungsform einer Vorrichtung zur Erzeugung eines Aerosols dargestellt. Dabei sind gleiche Teile mit den gleichen Bezugszeichen versehen wie in Fig. 1.

Die Vorrichtung weist in einem Zylinder 2a einen Verwirbelungsraum 2 auf, in dem auch die Gesamtmenge der zu verwirbelnden Substanz untergebracht ist. Die Volumenänderung des Verwirbelungsraums 2 erfolgt durch einen Kolben 2b, der in dem Zylinder 2a verschiebbar ist. Auf der dem Kolben 2b gegenüberliegenden Seite des Verwirbelungsraume ist ein Verschlußorgan 4 in Form eines Ventils vorgesehen.

Der Kolben 2b ist über eine aus dem Zylinder 2a heraus- und durch ein weiter unten zu erläuterndes Zählwerk 33 ragende Kolbenstange 34 mittels eines mit dieser verbundenen Griffes 34a im Zylinder 2a verschiebbar.

Der Kolben ist mit Dichtungen 35 versehen. Mit seiner zum Verwirbelungsraum 2 reichenden Stirnseite 36 ist eine flexible Hülle 37 in Form einer Latexmembran oder dergleichen verbunden, die weiter im Bereich des Verschlußorgans 4 bei 38 an der Wandung des Zylinders 2a festgelegt ist. Der Verwirbelungsraum 2 ist innerhalb der Hülle 37 ausgebildet. Die Substanz befindet sich ebenfalls innerhalb der Hülle. Hierdurch wird verhindert, daß sich Substanzmaterial an der Innenwandung des Zylinders 2a festsetzt und die Bewegung des Kolbens 2b erschwert oder sonstwie beeinträchtigt. Des weiteren ist in der Hülle 37 eine geerdete Schraubenfeder 5 angeordnet, welche zum einen an der Stirnseite 36 des Kolbens 2b, zum anderen im Bereich des Verschlußorgans 4 an der Wandung des Zylinders 2a festgelegt ist.

Über die dem Verwirbelungsraum 2 zugewandte Stirnseite 36 des Kolbens 2b ragt in den Verwirbelungsraum 2 ein Stößel 39, dessen Funktion weiter unten erläutert wird.

Das Verschlußorgan 4 weist einen beweglichen Ventilkörper 41 und einen Ventilsitz 42 auf. Der Ventilsitz 42 wird durch einen Dichtring 43 gebildet, der am Ventilkörper 41 im Schließzustand dicht aufliegt. Auf der dem Verwirbelungsraum 2 abgewandten Seite ist der Ventilkörper 41 mit einer konusförmigen Fläche 45 versehen, die beim erneuten Schließen des Ventils des Verschlußorgans 4 am Dichtring 43 entlanggleitet.

Die Innenseite des Dichtungsringes ist vorzugsweise nicht zylinderförmig, sondern schneidenförmig oder abgerundet ausgebildet.

Mit Abstand zur Dichtung 43 in Richtung auf den Verwirbelungsraum 2 hin ist ein ebenfalls elastischer Abstreifring 46 am Zylinder 2a festgelegt, der mit einem mit dem Ventilkörper 41 ebenfalls zum Verwirbelungsraum 2 hin verbundenen, flachen Zylinderkörper 47 mit gegenüber dem Ventilkörper 41 vergrößerten Durchmesser zusammenwirkt. Ventilkörper 41 und Zylinderkörper 47 können einstückig ausgebildet sein.

Im Zylinderkörper 47 ist eine zentralsymmetrische, kegelstumpfförmige Ausnehmung 48 ausgebildet, die mit einem kegelstumpfförmigen, vorderen Ende 49 des Stößels 39 in noch zu beschreibender Weise zusammenwirkt.

Mit dem Zylinder 2a ist ein Mundstück 51 verbunden, dessen Haupterstreckungsrichtung sich senkrecht zu der des Zylinders 2a erstreckt. Das Mundstück weist einen zu einer Auslaßöffnung 52 führenden Auslaßkanal 53 auf.

Zwischen Auslaßbereich 54 des Verwirbelungsraumes 2 hinter dem Verschlußorgan 4 und dem Auslaßkanal 53 ist eine Siebplatte 55 angeordnet, durch die das aus dem Verwirbelungsraum 2 herausgedrückte Aerosol in den Kanal 53 und von diesem aus der Öffnung 52 herausgedrückt wird, wobei die Aerosolströmung gleichgerichtet, d.h. in eine im wesentlichen laminare Strömung gebracht wird und durch die Verwirbelung im Verwirbelungsraum 2 entstandene Turbulenzen des Aerosols weitgehend unterdrückt werden.

In einer dem Verschlußorgan 4 abgewandten Trennwand 56 des Mundstücks 51 ist vorzugsweise fluchtend mit dem Verschlußorgan 4 eine Öffnung 57 ausgebildet, die durch eine Verschlußplatte 58 verschließbar ist, die über einen axialen Ansatz 59 des Ventilkörpers 41 beim Öffnen desselben verschließbar und bei geschlossenem Verschlußorgan 4 geöffnet ist.

Die Öffnung 57 mündet in einen Zwischenraum 60, der über auf ihrer Innenseite durch als Rückschlagventile 60a wirkende Klappen abdeckbare Öffnungen 60b in der Zylinderwand 2a mit der Umgebung in Verbindung steht. So kann über die Öffnungen 60b und die Öffnung 57 nur Luft eingesaugt, aber nicht herausgedrückt werden.

Wie schon oben erwähnt, ist an dem dem Mundstück 51 abgewandten Ende des Zylinders 2a ein Zählwerk 33 vorgesehen. Hierzu ist an der Kolbenstange 34 eine federnde Nase 61 ausgebildet, die auf ein Ritzel 62 wirkt, dessen Achse senkrecht zur Achse der Kolbenstange 34 ausgerichtet ist. An der abgewandten Stirnseite des Ritzels 62 ist eine Schnecke ausgebildet, die in einen großen Zahnkranz 63 mit innenliegender Verzahnung eingreift, dessen Achse parallel zur Achse der Kolbenstange 34 ausgerichtet ist und gegebenenfalls mit dieser zusammenfällt. An der Außenseite des Zahnkranzes 63 sind Markierungen angeordnet, die durch ein Fenster erkennbar sind.

Alternativ könnte ein Zahnriemen vorgesehen sein, der in gleicher Weise über einen federnden Ansatz an der Kolbenstange 34 gerichtet betätigbar ist und Farbmarkierungen aufweist, die durch ein Fenster festgestellt werden können. So kann in beiden Fällen festgestellt werden, ob die vorgesehene Anzahl von Betätigungen ausgeführt und damit die in der Vorrichtung ursprünglich eingefüllte Substanz verbraucht ist.

Dieses Ausführungsbeispiel der erfindungsgemäßen Vorrichtung funktioniert folgendermaßen:
Der Kolben 2b wird aus der in der Fig. 2A dargestellten Position, bei der er höchstens soweit nach oben, d.h. in Richtung auf das Mundstück 51, zugeschoben ist, daß die Spitze 49 des Ansatzes 39 in die konische Ausnehmung 48 der Platte 47 ragt, mittels des Griffes 34a nach unten, d.h. von dem Mundstück 51 fortgezogen. Das Verschlußorgan 4 ist dabei geschlossen. Hierdurch wird der Verwirbelungsraum 2 mit einem Unterdruck, einem Vakuum versehen. Gleichzeitig folgt die flexible Schraubenfeder 5 durch Auseinanderziehen derselben der Bewegung des Kolbens 2b.

Kurz bevor der Kolben 2b an der dem Mundstück 51 abgewandten Stirnseite des Zylinders 2a angelangt, befindet sich der zwischen dem Ventilkörper 41 und dem Ansatz 39 des Kolbens 2b befestigte zugfeste und auch gegen Zug relativ unelastische Faden 50 in seiner gespannten Stellung. Ein weiteres Herunterziehen des Kolbens 2b bis zur dem Mundstück 51 abgewandten Stirnseite bewirkt, daß das Verschlußorgan 4 durch Herunterziehen des Ventilkörpers 41 geöffnet wird (Fig. 2B). Gleichzeitig wird die Öffnung 57 durch die Verschlußplatte 58 verschlossen.

Durch das geöffnete Verschlußorgan 4 kann nun über die Öffnung 52 des Mundstücks Luft aufgrund des Unterdrucks des Verwirbelungsraums 2 in diesen einströmen, wodurch die in diesem befindliche Substanz verwirbelt wird und damit ein Aerosol entsteht. Dabei geben dann geladene Aerosolteilchen ihre Ladungen an der Oberfläche der geeignet geerdeten Schraubenfeder 5 ab, welche diese dann aus dem Verwirbelungsraum 2 heraus abführt.

Im folgenden wird mittels des Griffes 34a der Kolben 2b wieder auf das Mundstück 51 zugedrückt. Hierbei wird zunächst das im Verwirbelungsraum 2 gebildete Aerosol aus dem Verwirbelungsraum heraus und durch die Siebplatte 55 in den Kanal 53 des Mundstücks 51 gedrückt, bis die vordere Spitze 49 des Kolbenansatzes 39 in der Ausnehmung 48 der Platte 47 des Verschlußorgans 4 eingreift und das Verschlußorgan 4 wieder schließt. Gleichzeitig wird die Öffnung 57 geöffnet, indem die Verschlußplatte 58 von der Öffnung abgehoben wird.

Solange die Öffnung 57 geschlossen ist und damit das Verschlußorgan 4 geöffnet ist, kann der Benutzer der erfindungsgemäßen Vorrichtung nicht einatmen. Es wird damit verhindert, daß er eine undefinierte Menge an Aerosol aus dem Verwirbelungsraum 2 durch Einatmen einsaugt. Dadurch, daß während des Ausdrückens von Aerosol aus dem Verwirbelungsraum 2 und der Öffnungszeit des Verschlußorgans 4 die Öffnung 57 geschlossen ist, wird damit eine genau definierte Menge Aerosol aus dem Verwirbelungsraum 2 in das Mundstück 51 eingebracht, die durch den Hub des Kolbens 2b bestimmt ist. Wäre in diesem Zeitbereich die Öffnung 57 geöffnet, so könnte im Gegenstrom Luft durch die Öffnung 57 und das geöffnete Verschlußorgan 4 in den Verwirbelungsraum 2 einströmen, so daß ein Benutzer durch Einatmen eine undefinierte Menge Aerosol aus dem Verwirbelungsraum 2 einsaugen könnte.

Nachdem, wie beschrieben, die Öffnung 57 geöffnet wurde, kann der Benutzer das im Kanal 53 des Mundstücks 51 befindliche Aerosol inhalieren. Dabei kann Luft über die Öffnung 60b, den Raum 60 und die Öffnung 57 nachströmen, da sich beim Ansaugen die Klappen 60a von den Öffnungen 60b abheben und diese freigeben (wiederum Fig. 2A). Anschließend steht die erfindungsgemäße Vorrichtung zu einer erneuten Benutzung bereit.

## Patentansprüche

1. Vorrichtung zur Erzeugung eines Aerosols aus einer pulverförmigen Substanz, insbesondere zur Inhalation, mit einem die zu verwirbelnde Substanz aufnehmenden Verwirbelungsraum, in dem ein Unterdruck erzeugbar ist, in den durch eine verschließbare Öffnung ein Gas zum Verwirbeln der Substanz und zur Erzeugung eines Aerosols einbringbar und aus dem durch eine verschließbare Öffnung eine vorbestimmte Menge des Aerosols ausbringbar ist, dadurch gekennzeichnet, daß im Verwirbelungsraum (2) ein flexibles Element (5) aus elektrisch leitendem Material angeordnet ist.

2. Vorrichtung nach Anspruch 1, dadurch gekennzeichnet, daß sich das flexible Element (5) aus elektrisch leitendem Material über den gesamten Querschnitt des Verwirbelungsraumes (2) erstreckt.

3. Vorrichtung nach Anspruch 1 oder 2, dadurch gekennzeichnet, daß das Material Metall ist.

4. Vorrichtung nach einem der vorangehenden Ansprüche, dadurch gekennzeichnet, daß das Element (5) in Form eines Drahtknäuels ausgebildet ist.

5. Vorrichtung nach einem der Ansprüche 1 bis 3, dadurch gekennzeichnet, daß das Element (5) in Form einer mehrfach gewundenen Schraubendrahtfeder ausgebildet ist.

6. Vorrichtung nach einem der Ansprüche 1 bis 3, dadurch gekennzeichnet, daß das Element in Form einer räumlich auseinandergezogenen Drahtspirale (5) mit konischer oder doppelkonischer Kontur ausgebildet ist.

7. Vorrichtung nach einem der Ansprüche 1 bis 6, dadurch gekennzeichnet, daß der Verwirbelungsraum (2) in geschlossenem Zustand zur Herstellung des Unterdrucks in ihm vergrößerbar ist.

8. Vorrichtung nach Anspruch 7, dadurch gekennzeichnet, daß der Verwirbelungsraum (2) von einer Anordnung aus einem Kolben (2b) und einem Zylinder (2a) gebildet ist, bei der der Verwirbelungsraum (2) durch Verschieben des Kolbens (2b) gegenüber dem Zylinder (2a) vergrößerbar ist.

9. Vorrichtung nach einem der Ansprüche 1 bis 8, dadurch gekennzeichnet, daß das flexible Element (5) aus elektrisch leitendem Material an der dem Verwirbelungsraum (2) zugewandten Stirnseite (6, 36) des Kolbens (2b) an diesem sowie an dem der Stirnseite (6, 36) gegenüberliegenden Ende des Verwirbelungsraums (2) an der Wandung (7) des Zylinders (2a) festgelegt ist.

10. Vorrichtung nach einem der Ansprüche 1 bis 7, dadurch gekennzeichnet, daß der Verwirbelungsraum (2) durch einen an seinen Stirnseiten jeweils mit Deckplatten versehenen Faltenbalg gebildet und durch Ausdehnen des Faltenbalges vergrößerbar ist.

11. Vorrichtung nach einem der Ansprüche 1 bis 7 und 10, dadurch gekennzeichnet, daß das flexible Element aus elektrisch leitendem Material an den Deckplatten des Faltenbalges festgelegt ist.

12. Vorrichtung nach einem der vorangehenden Ansprüche, dadurch gekennzeichnet, daß Bereiche des Elements (5) zumindest an der Umfangswandung des Verwirbelungsraums (2) anliegen.
